(19) Europäisches Patentamt
European Patent Office
Office européen des brevets

(11) Publication number: 0 002 626 B1

# (12) EUROPEAN PATENT SPECIFICATION

(45) Date of publication of patent specification: 27.07.83

(21) Application number: 78300870.9

(22) Date of filing: 20.12.78

(51) Int. Cl.³: B 01 J 23/84, C 07 C 120/00, C 07 B 3/00, C 07 C 5/48

## (54) Ammoxidation of olefins with novel antimonate catalysts.

(30) Priority: 20.12.77 US 862268

(43) Date of publication of application: 27.06.79 Bulletin 79/13

(45) Publication of the grant of the patent: 27.07.83 Bulletin 83/30

(84) Designated Contracting States: BE CH DE FR GB LU NL SE

(56) References cited:
EP - A - 0 000 564
EP - A - 0 000 663
DE - A - 2 334 037
FR - A - 2 105 963
FR - A - 2 279 465
FR - A - 2 280 428
GB - A - 1 483 097
GB - A - 1 488 699
GB - B - 1 478 621
NL - A - 7 501 472
US - A - 3 409 697
US - A - 3 642 930
US - A - 3 803 204

(73) Proprietor: THE STANDARD OIL COMPANY
Midland Building
Cleveland, Ohio 44115 (US)

(72) Inventor: Suresh, Dev Dhanaraj
1052 Iroquois Run
Madeconia Ohio 44056 (US)
Inventor: Grasselli, Robert Karl
150 Greentree Road
Chagrin Falls Ohio 44022 (US)
Inventor: Orndoff, David Allan
5519 Warner Hollow Road
Windsor Ohio 44099 (US)

(74) Representative: Smith, Sydney et al,
Elkington and Fife High Holborn House 52/54
High Holborn
London WC1V 6SH (GB)

(56) References cited:
US - A - 3 836 586
US - A - 3 911 039
US - A - 3 925 255
US - A - 4 012 449
US - A - 4 036 901

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 002 626 B1

Courier Press, Leamington Spa, England.

# Ammoxidation of Olefins with novel antimonate catalysts

The present invention relates to a novel catalyst and process for the catalytic vapour phase ammoxidation of olefins to nitriles using antimonate catalysts.

U.S. 3,716,496 discloses a catalyst useful for the ammoxidation of olefins to nitriles having the formula

$$Fe_{10}Sb_{20-60}Me_{0.01-1}Te_{0.05-5}Q_{0.1-20}O_x$$

wherein Me is an element selected from the group consisting of V, Mo, and W and

Q is an element selected from the group consisting of Cu, Ag, Be, Mg, Ca, Sr, Ba, Zn, Cd, La, Ce and Al.

Netherlands Patent 7,501,472 discloses similar catalysts, these catalysts also containing at least one of P and B.

GB—A—1,488,699 discloses an improved method of making a catalyst composition containing the elements antimony and uranium and optionally other catalytic components such as iron, bismuth, molybdenum, tungsten, nickel and cobalt for use in the catalytic oxidation and/or ammoxidation of olefins.

In our FR—A—2,105,963 there is disclosed a process for the preparation of maleic anhydride by the oxidation of paraffins in the presence of a catalyst comprising antimony, molybdenum and at least one element selected from iron and vanadium.

In our US—A—3,409,697 there is described the use of antimony oxide-iron oxide base catalyst compositions of the general formula

$$Sb_aFe_bO_c$$

wherein a is 1 to 99, b is 50 to 1 and c is a number taken to satisfy the average valencies of antimony and iron in the oxidation states in which they exist in the catalyst as defined by the empirical formula above, which contain promoters in the oxidative dehydrogenation in the vapour phase of a monoolefin to a diolefin. Promoter elements include bismuth, copper, tin, germanium, uranium, rhenium, niobium, silver, cerium, tellurium, tungsten, manganese, gallium, lead, tantalum, palladium, cadmium, sirconium, thorium, vanadium, nickel, titanium, molybdenum, zinc, barium, calcium, thallium, arsenic and rhodium.

In commercial vapour phase processes for producing acrylonitrile, a liquid reaction product containing acrolein, acrylic acid and acetonitrile as well as acrylonitrile is normally obtained. It is often difficult to separate acrolein, acrylic acid and/or acetonitrile from this liquid reaction product, and hence processes yielding these unwanted liquid by-products in significant amounts are disadvantageous commercially. Unfortunately, vapour phase ammoxidation processes using promoted $Fe\text{-}Te\text{-}Sb\text{-}O_x$ catalysts do yield these unwanted by-products in significant amounts. Furthermore, tellurium in such catalysts tends to volatilize as tellurium oxide, which leads not only to a significant decrease in catalytic activity with time but also to significant pollution problems.

It is therefore an object of the present invention to provide a process for the ammoxidation of olefins to nitriles in which the production of acrolein, acrylic acid and acetonitrile is significantly reduced by means of a novel catalyst not containing tellurium as an essential component.

Thus, the present invention therefore provides a novel catalyst which comprises an oxide complex of the following general formula

$$Fe_{1-50}Bi_{0.1-20}Sb_{1-100}A_aB_bC_cO_x$$

in which

A represents Cu and/or Cr;

B represents alkali metal, alkaline earth metal, Co, Ni, Zn, Cd, a rare earth, Ga, Tl and/or Th;

C represents Mo, W, Nb and/or Ta;

a is 0.01—20;

b is 0—20;

c is 0.01—20; and

x is a number determined by the oxygen requirements of the other elements present.

In addition, the present invention also provides a novel ammoxidation process in which an olefin together with oxygen and ammonia is contacted with a catalyst at elevated temperature, characterised in that the catalyst is a catalyst of the above composition.

In accordance with another aspect of the present invention it has also been found that various aldehydes and acids can be ammoxidized with the catalysts according to the invention, and thus the present invention also provides ammoxidation processes of the type described above characterised in that the feed comprises an aldehyde and/or alcohol rather than an olefin.

Finally it has also been found that the catalysts of the present invention are effective in the oxidation of various olefins to oxygenated compounds and the oxydehydrogenation of various olefins to diolefins. Thus, the present invention provides two additional processes for the oxidation and oxydehydrogenation of olefins in which the olefin to be reacted together with oxygen is contacted with a catalyst characterised in that the catalyst is a catalyst of the formula given above.

The novel catalysts of the present invention are particularly useful in the ammoxidation of olefins to nitriles. They can also be used, however, in the ammoxidation of alcohols and aldehydes to nitriles and in the oxidation of olefins to oxygenated compounds and the oxidative dehydrogenation of olefins to diolefins and aromatics.

Ammoxidation

A wide variety of different reactants can be ammoxidized according to the present invention to produce nitriles. For example, olefins such as propylene and isobutylene, alcohols such as t-butyl alcohol and aldehydes such as acrolein and methacrolein can be readily converted to nitriles according to the present invention. In general, compounds which can be converted to nitriles by the ammoxidation reaction according to the invention include 3 to 9 carbon atom hydrocarbons, which may be unsubstituted or substituted with oxygen or hydroxyl groups. Preferred starting materials are olefins, aldehydes and alcohols containing 3 or 4 carbon atoms.

The general ammoxidation process for converting olefins, alcohols and aldehydes to nitriles is well known, and is described, for example, in U.S. Patent No. 3,546,138. In general, the ammoxidation reaction is accomplished by contacting the reactant, oxygen and ammonia with a particular catalyst in the vapour phase. The reaction according to the invention is carried out in the same manner and under the conditions generally set forth in this patent.

In a preferred embodiment, the process according to this invention comprises contacting a mixture comprising propylene or isobutylene, ammonia and oxygen with the promoted catalyst of this invention at an elevated temperature and at atmospheric or near atmospheric pressure.

Any oxygen source may be employed in this process, for reasons of economy, however, it is preferred that air is employed as the oxygen source. From a purely technical viewpoint, relatively pure molecular oxygen will given equivalent results. The molar ratio of oxygen to olefin in the feed to the reaction vessel should be in the range of 0.5:1 to 4:1 and a ratio of from 1:1 to 3:1 is preferred.

Low molecular weight saturated hydrocarbons do not appear to influence the reaction to an appreciable degree, and these materials can be present; consequently, the addition of saturated hydrocarbons to the feed to the reaction is contemplated within the scope of this invention. Similarly, diluents, such as nitrogen and the oxides of carbon, may be present in the reaction mixture without harmful effect.

The molar ratio of ammonia to olefin in the feed to the reactor may vary between about 0.05:1 to 5:1. There is no real upper limit for the ammonia/olefin ratio, but there is generally no reason to exceed the 5:1 ratio. At ammonia/olefin ratios appreciably less than the stoichiometric ratio of 1:1, varying amounts of oxygenated derivatives of the olefin will be formed.

Significant amounts of unsaturated aldehydes, as well as nitriles, will be obtained at ammonia-olefin ratios substantially below 1:1, i.e., in the range of 0.15:1 to 0.75:1. Above the upper limit of this range, the amount of aldehydes produced rapidly decreases. Within the ammonia-olefin range stated, maximum utilisation of ammonia is obtained and this is highly desirable. It is generally possible to recycle any unreacted olefin and unconverted ammonia.

Water can also be included in the feed although it is not essential. In some cases, for example, in fixed-bed systems, water may improve the selectivity of the reaction and the yield of nitrile. However, reactions not including water in the feed are also within the scope of the present invention.

In general, the molar ratio of added water to olefin, when water is added, is in the neighbourhood of 0.1:1 or higher. Ratios on the order of 1:1 to 3:1 are particularly desirable, but higher ratios may be employed, suitably up to about 10:1.

The reaction is carried out at an elevated temperature such as 200—600°C, preferably 400—500°C. The pressure at which the reaction is conducted is also an important parameter, and the reaction should be carried out at about atmospheric or slightly above atmospheric (2 to 3 atmospheres) pressure. In general, high pressures, i.e. above 15 atmospheres, are not suitable since higher pressures tend to favour the formation of undesirable by-products.

The apparent contact time is not critical, and contact times in the range of from 0.1—50 seconds may be employed. The optimal contact time will, of course, vary depending upon the reactant being used, but in general a contact time of from 1—15 seconds is preferred.

The ammoxidation reaction according to the invention is carried out in the vapour phase. The process is normally conducted on a continuous basis using either a fixed-bed or a fluid-bed catalyst. However, a batch operation can be employed.

The reaction product passing out of the reactor is normally in the form of a gas. Conventionally, this gaseous reaction product is treated to remove ammonia and then partially condensed either by indirect contact with a cooling medium or direct contact with water to form a liquid phase containing acrylonitrile, acrolein, acrylic acid, hydrogen cyanide and acetonitrile and a vapour phase containing

carbon dioxide, carbon monoxide, nitrogen and water. The acrylonitrile is then separated from the liquid phase by a number of different techniques such as, for example, distillation or water extraction/distillation. Additional steps can be employed to recover separately acrylic acid and/or acrolein from the gross reaction product.

In accordance with the present invention, the amount of acrolein, acrylic acid and acetonitrile produced by the ammoxidation reaction according to the invention are significantly reduced. Thus, separation of the acrylonitrile from the remaining components of the crude reaction product is simplified. Further, problems associated with disposal of the unwanted by-products, such as water pollution, are greatly reduced.

Oxidation

As previously indicated, the catalysts according to this invention can also be used in the catalytic oxidation of olefins to various different reaction products.

The reactants used in the oxidation to oxygenated compounds are oxygen and an olefin such as propylene, isobutylene and other olefins having up to three contiguous carbon atoms (i.e. three carbon atoms arranged in a straight chain).

The olefins may be in admixture with paraffinic hydrocarbons, such as ethane, propane, butane and pentane; for example, a propylene-propane mixture may constitute the feed. This makes it possible to use ordinary refinery streams without special preparation.

The temperature at which this oxidation is carried out may vary considerably depending upon the catalyst, the particular olefin being oxidized and the correlated conditions of the rate of throughput or contact time and the ratio of olefin to oxygen. In general, when operating at pressures near atmospheric, i.e., 0.1 to 10 atmospheres, temperatures in the range of 150°C to 600°C may be advantageously employed. However, the process may be conducted at other pressures, and in the case where superatmospheric pressures, e.g., above 10 atmospheres are employed, somewhat lower temperatures are possible. In the case where this process is employed to convert propylene to acrolein, a temperature range of 200°C to 500°C has been found to be optimum at atmospheric pressure.

While pressures other than atmospheric may be employed, it is generally preferred to operate at or near atmospheric pressure, since the reaction proceeds well at such pressures and the use of expensive high pressure equipment is avoided, and formation of undesired by-products and waste is diminished.

The apparent contact time employed in the process is not critical and it may be selected from a broad operable range which may range from 0.1 to 50 seconds. The apparent contact time may be defined as the length of time in seconds which a unit volume of gas measured under the conditions of reaction is in contact with the apparent unit volume of the catalyst. It may be calculated, for example, from the apparent volume of the catalyst bed, the average temperature and pressure of the reactor, and the flow rates of the several components of the reaction mixture.

The optimum contact time will, of course, vary depending upon the olefin being treated, but in the case of propylene and isobutylene, the preferred contact time is 0.15 to 15 seconds.

A molar ratio of oxygen to olefin of from 0.5:1 to 5:1 generally gives the most satisfactory results. For the conversion of propylene to acrolein, a preferred ratio of oxygen to olefin is from about 1:1 to about 2:1. The oxygen used in the process may be derived from any source; however, air is the least expensive source of oxygen and is preferred for that reason.

The addition of water to the reaction mixture in oxidation reactions can have a beneficial influence on the conversion and yields of the desired product, especially in fixed-bed reactions. The manner in which water affects the reaction is not fully understood. In any event, it is preferred in fixed-bed operation to include water in the reaction mixture, and in general a ratio of olefin to water in the reaction mixture of from 1:0.25 to 1:10 will give very satisfactory results while a ratio of 1:0.5 to 1:6 has been found the optimum when converting propylene to acrolein.

Inert diluents such as oxygen and carbon dioxide, may be present in the reaction mixture.

Oxydehydrogenation

In accordance with the present invention, the promoted catalyst system of the present invention can also be employed in the catalytic oxidative dehydrogenation of olefins to diolefins and aromatic compounds. In this process, the feed stream in vapour form containing the olefin to be dehydrogenated and oxygen is conducted over the promoted catalyst at a comparatively low temperature to obtain the corresponding diolefin.

By the term "olefin" as used herein is meant open chain as well as cyclic olefins. The olefins dehydrogenated according to this invention have at least four and up to nine non-quaternary carbon atoms, of which at least four are arranged in series in a straight chain or ring. The olefins are preferably either normal straight chain or tertiary olefins. Both cis and trans isomers, where they exist, can be dehydrogenated.

Among the many olefinic compounds which can be dehydrogenated in this way are butene-1; butene-2; pentene-1; pentene-2; pentenes, hexenes, etc. such as 2-methylpentene-1, 3-methylbutene-1, 3,4-dimethyl-pentene-1, 4-methyl-pentene-2; heptene-1; octene-1; cyclopentene; cyclohexene; 3-methyl cyclohexene and cycloheptene.

Open chain olefins yield diolefins, and, in general, six-membered ring olefins yield aromatic ring compounds. The higher molecular weight open chain olefins may cyclize to aromatic ring compounds.

The feed stock in addition to the olefin and oxygen may contain one or more paraffinic or naphthenic hydrocarbons having up to about ten carbon atoms, which may be present as impurities in some petroleum hydrocarbon stocks and which may also be dehydrogenated in some cases.

The amount of oxygen is preferably within the range of from 0.3 to 4 moles per mole of double-bond created. Stoichiometrically, 0.5 mole of oxygen is required for the dehydrogenation of one mole of a monoolefin to a diolefin. It is preferred to employ an excess of oxygen, e.g. an oxygen/olefin ratio of from 0.6 to 3, in order to ensure a higher yield of diolefin per pass. The oxygen can be supplied as pure or substantially pure oxygen or as air or in the form of hydrogen peroxide.

When pure oxygen is used, it may be desirable to incorporate a diluent in the mixture such as steam, carbon dioxide or nitrogen.

The feed stock is preferably catalytically dehydrogenated in the presence of steam, but this is not essential. Usually, from 0.1 to 6 moles of steam per mole of olefin reactant is employed, but amounts larger than this can be used.

The dehydrogenation proceeds at temperatures preferably within the range of from 300°C to 1000°C. Optimum yields are obtainable at temperatures within the range from 400 to 550°C. However, since the reaction is exothermic, temperatures in excess of 550°C should not be used, unless means are provided to carry off the heat liberated during the reaction. Due to the exothermic nature of the reaction, the temperature of the gaseous reaction mixture will be higher than the temperature of the feed entering the system by as much as 75°C. The temperatures referred to are those of the entering gas feed near the reactor inlet.

The preferred reaction pressure is approximately atmospheric, preferably within the range of from about 0.1 to about 5 atmospheres.

Only a brief contact time with the catalyst is required for effective dehydrogenation. The apparent contact time with the catalyst can vary from about 0.5 up to about 50 seconds but higher contact times can be used if desired. At these contact times, comparatively small reactors and small amounts of catalyst can be used effectively.

In carrying out the foregoing processes, any apparatus of the type suitable for carrying out oxidation reactions in the vapour phase may be employed. The processes may be conducted either continuously or intermittently. The catalyst may be a fixed-bed employing a large particulate or pelleted catalyst or a fluid-bed catalyst may be employed.

The catalysts according to the present invention are oxide complexes of iron, bismuth and antimony promoted with various additional elements and are described by the following general formula:

$$Fe_{1-50}Bi_{0.1-20}Sb_{1-100}A_aB_bC_cO_x$$

wherein

A represents Cu and/or Cr;

B represents an alkali metal, alkaline earth metal, Co, Ni, Zn, Cd, rare earth, Ga, Tl and/or Th;

C represents Mo, W, Nb and/or Ta.

a is 0.01—20;

b is 0—20;

c is 0.01—20; and

x is a number determined by the oxygen requirements of the other elements present.

Catalysts in which C is a mixture of Mo and W are especially preferred.

These catalysts can be used either in unsupported form or supported on suitable carriers such as $SiO_2$, $Al_2O_3$, $BPO_4$, $SbPO_4$, $ZrO_2$ and Alundum. The catalyst can also be coated on these supports by special techniques known in the art.

These catalysts can be prepared by conventional techniques. In this regard, U.S. Patent No. 3,546,138, referred to above, teaches how catalysts of this type can be made. U.S. Patents Nos. 3,461,150; 3,325,504; 3,933,751; 3,435,061; 3,431,292 and 3,308,151 also disclose how catalysts of this general type can be made.

Examples

In order to more thoroughly describe the present invention, the following working examples in which propylene was ammoxidized to acrylonitrile are presented. In these examples, the term "% yield" means

$$\frac{\text{moles product formed}}{\text{moles reactant fed}} \times 100$$

Examples 1—3—50% $W_{0.2}Mo_1Bi_2Cu_3Fe_{12}Sb_{25}O_x$+50% $SiO_2$

A catalyst of the above formula was prepared by the following procedure.

96.96 grams $Fe(NO_3)_3 \cdot 9H_2O$, 1.08 grams $(NH_4)_6W_7O_{24} \cdot 6H_2O$, 3.53 grams $(NH_4)_6Mo_7O_{24} \cdot 4H_2O$, 19.40 grams $Bi(NO_3)_3 \cdot 5H_2O$ and 14.50 grams $Cu(NO_3)_2 \cdot 3H_2O$ were individually dissolved in small amounts of water at 60°C with stirring. The individual solutions were then added together in the order given above, i.e. the W-containing solution was first added to the Fe-containing solution, then the Mo solution was added to the Fe/W solution, and so forth until an aqueous solution containing Fe, W, Mo, Bi and Cu was obtained. To this salt solution was added 72.87 grams $Sb_2O_3$ which had been slurried in about 400 ml concentrated $HNO_3$ at 60—70°C for 1½ hours. Sufficient reagent grade $NH_4OH$ (about 150 cc) was then added to the mixture to adjust the pH to 2.0 and the mixture was then evaporated to dryness. The dried precipitation was heat treated in air for 3 hours at 290°C and then for 3 more hours at 425°C. The resultant material was then screened to 20—35 mesh to produce the objective catalyst.

5 c.c.s of this catalyst was charged into a fixed-bed reactor, and a feed comprising 1 propylene/1.1 $NH_3$/10.6 air/4$H_2O$ was fed to the reactor at elevated temperature. The reaction temperature and the contact time were varied from example to example. The gross reaction product was recovered and analyzed. The results obtained are set forth in the following Table I.

TABLE I

$W_{0.2}Mo_1Bi_2Cu_3Fe_{12}Sb_{25}O_x$+50% $SiO_2$

| Example | Contact time (sec) | Reaction temp. °C | % Yield: Unreacted propylene | % Yield: Acrylonitrile |
|---|---|---|---|---|
| 1 | 3 | 430 | 11.8 | 67.0 |
| 2 | 6 | 430 | 5.1 | 70.8 |
| 3 | 3 | 460 | 3.9 | 73.5 |

| Example | % Yield: Acrolein | Acrylic acid | Acetonitrile | HCN | CO | $CO_2$ | % AN Selectivity |
|---|---|---|---|---|---|---|---|
| 1 | — | 0.3 | 1.1 | 5.5 | 4.8 | 9.6 | 75.9 |
| 2 | — | 1.8 | 0.4 | 7.8 | 5.5 | 8.7 | 74.5 |
| 3 | — | 0.3 | 0.4 | 6.9 | 4.6 | 10.5 | 76.5 |

As will be noted from the foregoing experiments, acrylonitrile was produced in high yields with good selectivities. Moreover, the amounts of unwanted by-products acrolein, acrylic acid and acetonitrile were very small. Thus, it will be appreciated that the present invention marks a significant improvement over many known processes for producing acrylonitrile which produce unwanted by-products in significant amounts.

Examples 4—10

Additional experiments in which propylene was ammoxidized to acrylonitrile were conducted. These experiments were carried out in the same way as those of Examples 1 to 3 except that the feed consisted of 1.0 propylene/1.1$NH_3$/10.6 air/4.0 $H_2O$, the contact time was 3 seconds and the catalyst used was different. The compositions of the various catalysts used in these experiments as well as the results obtained are set forth in the following Table II.

TABLE II

| Example | Catalyst | Reaction Temp. °C. |
|---|---|---|
| 4 | $Fe_{12}Sb_{25}Cr_{0.2}Bi_2Cu_3Mo_1O_x$+40% $SiO_2$ | 460 |
| 5 | $Fe_{12}Sb_{25}Cr_{0.2}Bi_2Cu_3Mo_1O_x$+40% $SiO_2$ | 430 |
| 6 | $Fe_{12}Sb_{27}W_{0.2}Bi_2Cu_5Mo_1O_x$+40% $SiO_2$ | 460 |
| 7 | $Fe_{12}Sb_{25}W_{0.2}Bi_2Cu_3Mo_3O_x$+40% $SiO_2$ | 460 |
| 8 | $Fe_{12}Sb_{25}W_{0.2}Bi_2Cu_3Mo_3O_x$+40% $SiO_2$ | 430 |

TABLE II (Cont'd)

| | % Propylene conversion to | | | | | | | | |
|---|---|---|---|---|---|---|---|---|---|
| Example | Unreacted propylene | Acrylo-nitrile | Acrolein | Acrylic acid | Aceto-nitrile | HCN | CO | $CO_2$ | %AN selectivity |
| 4 | 12.2 | 60.2 | 9 | 0.5 | 1.9 | 0.3 | 1.2 | 23.7 | 68.6 |
| 5 | 16.5 | 57.6 | 0 | 0.2 | 1.9 | 0.4 | 1.1 | 22.2 | 69.0 |
| 6 | 22.1 | 43.1 | 3.5 | 0.2 | 2.3 | 0.3 | 0.4 | 28.1 | 55.4 |
| 7 | 47.8 | 20.5 | 3.7 | 0.4 | 1.8 | 0.3 | 6.3 | 19.2 | 39.3 |
| 8 | 39.3 | 29.2 | 0 | 1.1 | 5.1 | 5.1 | 9.6 | 10.6 | 48.2 |

## Claims

1. A catalyst comprising an oxide complex of the following general formula:

$$Fe_{1-50}Bi_{0.1-20}Sb_{1-100}A_aB_bC_cO_x$$

in which

A represents Cu and/or Cr;
B represents an alkali metal, alkaline earth metal, Co, Ni, Zn, Cd, rare earth, Ga, Tl and/or Th;
C represents Mo, W, Nb and/or Ta;
a is 0.01—20;
b is 0—20;
c is 0.01—20; and
x is a number determined by the oxygen requirements of the other elements present.

2. A catalyst as claimed in claim 1 characterised in that it contains Mo and/or W.

3. A catalyst as claimed in claim 1 or claim 2 characterised in that *b* is greater than 0.

4. An ammoxidation process for producing a nitrile in which an olefin, an aldehyde or an alcohol is contacted together with oxygen and ammonia in the vapour phase with a catalyst at elevated temperature, characterised in that the catalyst is a catalyst as claimed in any of claims 1 to 3.

5. An oxidation process for oxidizing an olefin in which an olefin having no more than three contiguous carbon atoms and oxygen are contacted at elevated temperature and in the vapour phase with a catalyst, characterised in that the catalyst is a catalyst as claimed in any of claims 1 to 3.

6. An oxydehydrogenation process wherein an olefin having from four to up to 9 non-quaternary carbon atoms at least four of which are arranged in series in a straight chain or in a ring and oxygen are contacted at elevated temperature in the vapour phase with a catalyst characterised in that the catalyst is a catalyst as claimed in any of claims 1 to 3.

## Patentansprüche

1. Katalysator, bei dem es sich handelt um einen Oxid-komplex der folgenden allgemeinen Formel:

$$Fe_{1-50}Bi_{0.1-20}Sb_{1-100}A_aB_bC_cO_x$$

worin bedeuten:

A Cu und/oder Cr;

B ein Alkalimetall, ein Erdalkalimetall, Co, Ni, Zn, Cd, ein Element der Seltenen Erden, Ga, Tl und/oder Th;

C Mo, W, Nb und/oder Ta;

a 0,01 bis 20;

b 0 bis 20;

c 0,01 bis 20; und

x eine Zahl, die durch den Sauerstoffbedarf der übrigen vorhandenen Elemente bestimmt wird.

2. Katalysator nach Anspruch 1, dadurch gekennzeichnet, daß er Mo und/oder W enthält.

3. Katalysator nach Anspruch 1 oder 2, dadurch gekennzeichnet, daß b größer als 0 ist.

4. Ammoxidationsverfahren zur Herstellung eines Nitrils, bei dem ein Olefin, ein Aldehyd oder ein Alkohol zusammen mit Sauerstoff und Ammoniak in der Dampf- bzw. Gasphase bei erhöhter Temperatur mit einem Katalysator in Kontakt gebracht wird, dadurch gekennzeichnet, daß es sich bei dem Katalysator um einen Katalysator nach einem der Ansprüche 1 bis 3 handelt.

5. Oxidationsverfahren zum Oxidieren eines Olefins, bei dem ein Olefin mit nicht mehr als drei aneinandergrenzenden Kohlenstoffatomen und Sauerstoff bei erhöhter Temperatur und in der Dampf- bzw. Gasphase mit einem Katalysator in Kontakt gebracht werden, dadurch gekennzeichnet, daß es sich bei dem Katalysator um einen Katalysator nach einem der Ansprüche 1 bis 3 handelt.

6. Oxydehydrierungsverfahren, bei dem ein Olefin mit vier bis zu neun nicht-quaternären Kohlenstoffatomen, von denen mindestens vier in einer geraden Kette oder in einem Ring in Reihe hintereinander angeordnet sind, und Sauerstoff bei erhöhter Temperatur in der Dampf- bzw. Gasphase mit einem Katalysator in Kontakt gebracht werden, dadurch gekennzeichnet, daß es sich bei dem Katalysator um einen Katalysator nach einem der Ansprüche 1 bis 3 handelt.

**Revendications**

1. Catalyseur comprenant un oxyde complexe de la formule générale suivante:

$$Fe_{1-50}Bi_{0.1-20}Sb_{1-100}A_aB_bC_cO_x,$$

dans laquelle

A représente Cu et/ou Cr;

B représente un métal alcalin, un métal alcalinoterreux, Co, Ni, Zn, Cd, une terre rare, Ga, Tl et/ou Th;

C représente Mo, W, Nb et/ou Ta;

a a une valeur de 0,01 à 20;

b a une valeur de 0 à 20;

c a une valeur de 0,01 à 20; et

x est un nombre déterminé par les exigences en oxygène des autres éléments présents.

2. Catalyseur selon la revendication 1, caractérisé par le fait qu'il contient Mo et/ou W.

3. Catalyseur selon la revendication 1 ou la revendication 2, caractérisé par le fait que *b* est plus grand que 0.

4. Procédé d'ammoxydation pour produire un nitrile dans lequel une oléfine, un aldéhyde ou un alcool est mis en contact en même temps que de l'oxygène et de l'ammoniac dans la phase vapeur avec un catalyseur à température élevée, caractérisé par le fait que le catalyseur est un catalyseur selon l'une des revendications 1 à 3.

5. Procédé d'oxydation pour oxyder une oléfine dans lequel une oléfine n'ayant pas plus de trois atomes de carbone contigus et de l'oxygène sont mis en contact à température élevée et dans la phase vapeur avec un catalyseur, caractérisé par le fait le catalyseur est un catalyseur selon l'une des revendications 1 à 3.

6. Procédé d'oxydéshydrogénation dans lequel une oléfine ayant de quatre à neuf atomes de carbone non-quaternaires dont au moins quatre sont disposés en série dans une chaîne droite ou dans un cycle et de l'oxygène sont mis en contact à température élevée dans la phase vapeur avec un catalyseur, caractérisé par le fait que le catalyseur est un catalyseur selon l'une des revendications 1 à 3.